# EUROPEAN PATENT APPLICATION

(11) **EP 3 269 418 A1**
(43) Date of publication of application: **17.01.2018**
(21) Application number: 16305892.8
(22) Date of filing: 12.07.2016
(51) Int. Cl.: A61M 39/20, A61M 5/31

(54) **TIP CAP ASSEMBLY, MEDICAL INJECTION SYSTEM AND PROCESS FOR PRODUCING A MEDICAL INJECTION SYSTEM**

(71) Applicant: Becton Dickinson France, 38800 Le Pont-de-Claix (FR)
(72) Inventor: RIVIER, Cédric, 38340 VOREPPE (FR); ALVAIN, Olivier, 38180 SEYSSINS (FR)
(74) Representative: Regimbeau

(57) **Abstract**

The invention relates to a tip cap assembly (40) adapted to close a fluid passageway (27) extending through a tip (23) of a container (20) of a medical injection system (10), the tip cap assembly (40) including: a rigid outer cap (41) including an inner skirt (44) extending proximally and having an outer threaded surface (44a), an elastomeric inner cap (42) arranged within the outer cap (41), the inner cap (42) having a protrusion (42a) extending proximally and adapted to close the fluid passageway (27), characterized in that the outer cap (41) further includes an outer skirt (45) extending proximally and around the inner skirt (44) so as to define an annular groove (46) between the inner skirt (44) and the outer skirt (45), and that the outer skirt (45) includes a proximal member (48) having a projection extending inwardly or a recess depressed outwardly.

## Description

### FIELD OF THE INVENTION

The present invention relates to a medical injection system for use in delivering a pharmaceutical composition, vitamins, a vaccine or any other type of medical solution to a body of an intended patient, and also relates to a tip cap assembly adapted to be used with such a medical injection system. The invention further relates to a process for producing such a medical injection system.

In this application, the distal end of a component or apparatus must be understood as meaning the end furthest from the hand of the user and the proximal end must be understood as meaning the end closest to the hand of the user, with reference to the injection system intended to be used with said component or apparatus. As such, in this application, the distal direction must be understood as the direction of injection with reference to the injection system, and the proximal direction is the opposite direction, i.e. the direction towards the hand of the user.

### BACKGROUND OF THE INVENTION

A medical injection system includes a container for securely storing medical solution therein. Such a container generally includes an elongated hollow body and a tip extending distally from the body. The tip of the container is provided with a fluid passageway extending through the tip, so as to allow the medical solution in the container to be administered to a patient as necessary.

WO 2015/055608 describes an injection system including a longitudinal barrel, a distally projecting tip, a collar engaged around the distally projecting tip, and a tip cap assembly. The tip cap assembly includes an elastomeric inner cap having a frusto-conical protrusion, and a rigid outer cap which can be securely disposed around the elastomeric inner cap. The collar has an inner thread designed to cooperate with an outer thread of the outer cap of the tip cap assembly.

The injection system is configured such that when the tip cap assembly is fully assembled to the collar, a proximal surface of the outer cap comes in contact with a distal surface of the collar. According to the configuration, however, force applied in an attempt to tighten the tip cap assembly may be directly transmitted to the distal surface of the collar. As a result, a crack(s) may be formed on the collar due to excessive mechanical stress generated during or after the assembly. Such a crack(s) may lead to a poor connection of the adaptor with an intravenous (IV) line or a needle hub and therefore may lead to contamination of the medical solution contained in the barrel or result in unintended exposure of a medical practitioner or patient to the medical solution.

### SUMMARY OF THE INVENTION

Accordingly, an object of the present invention is to provide a medical injection system with a tip cap assembly which can eliminate or reduce the risk of forming a crack(s) on the collar due to excessive mechanical stress.

According to an embodiment of the invention, a tip cap assembly adapted to close a fluid passageway extending through a tip of a container of a medical injection system is provided. The tip cap assembly comprises a rigid outer cap comprising an inner skirt extending proximally and having an outer threaded surface, and an elastomeric inner cap arranged within the outer cap, the inner cap having a protrusion extending proximally and adapted to close the fluid passageway. The outer cap further comprises an outer skirt extending proximally and around the inner skirt so as to define an annular groove between the inner skirt and the outer skirt. The outer skirt comprises a proximal member having a projection extending inwardly or a recess depressed outwardly.

According to an embodiment of the invention, the proximal member may be connected to the outer skirt by a weakened portion.

According to an embodiment of the invention, the proximal member, the outer skirt and the weakened portion of the outer cap may be integrally formed with each other.

According to an embodiment of the invention, the inner skirt may extend beyond the outer skirt in a proximal direction.

According to an embodiment of the invention, a medical injection system is provided. The medical injection system comprises a container including a longitudinal barrel, a tip projecting distally from the barrel and provided with a fluid passageway extending through the tip, and a collar extending around the tip, the collar having an internal threaded surface facing an external surface of the tip, and a tip cap assembly configured in accordance with any one of the embodiments described above. The tip cap assembly is screwed onto the collar in cooperation between the outer threaded surface of the outer cap and the internal threaded surface of the collar so as to close the fluid passageway. The proximal member of the outer skirt retains the outer cap onto the collar at a predetermined position relative to each other. A longitudinal gap is provided between a distal end of the collar and a bottom of the groove when the outer cap is retained onto the collar by the proximal member.

According to an embodiment of the invention, the collar may have a complementary shape to the projection or to the recess formed on the proximal member of the outer skirt.

According to an embodiment of the invention, the collar may have a recess or protrusion engaged with the protrusion or the recess of the proximal member.

According to an embodiment of the invention, the collar and the container may be integrally formed with each other.

According to an embodiment of the invention, an inner surface of the inner skirt of the outer cap may be in contact with the external surface of the tip of the container.

According to an embodiment of the invention, the proximal member may have an annular shape around the collar.

According to an embodiment of the invention, a process for producing a medical injection system is provided. The medical injection system comprises a container formed with a fluid passageway extending through a distal tip of the container and a tip cap assembly adapted to close the fluid passageway. The container comprises a collar extending around the tip. The tip cap assembly comprises an inner skirt extending proximally and an outer skirt extending around the inner skirt so as to form an annular groove between the inner skirt and the outer skirt. The process comprises screwing the tip cap assembly onto the collar such that the collar is introduced to the groove until the tip cap assembly reaches a predetermined position relative to the collar so as to form a longitudinal gap between a distal end of the collar and a bottom of the groove.

### BRIEF DESCRIPTION OF THE FIGURES

The present invention will be described in further details in the following description with reference to the accompanying Figures, in which:
Figure 1 shows a medical injection system according to an embodiment of the invention;
Figure 2 is a longitudinal sectional view showing the medical injection system;
Figure 3 is an enlarged view showing a distal end of the medical injection system;
Figure 4 shows a tip cap assembly of the medical injection system without an inner cap; and
Figure 5 is a longitudinal sectional view showing the tip cap assembly without the inner cap.

### DETAILED DESCRIPTION OF EMBODIMENTS OF THE INVENTION

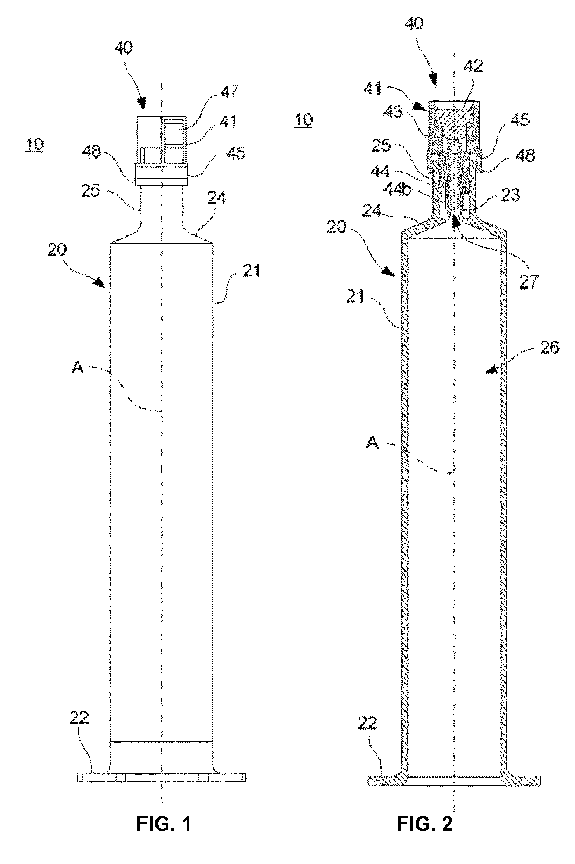

Figures 1 and 2 show a medical injection system 10 according to an embodiment of the invention. The medical injection system 10 will be described as configured to have a luer lock type fitting by way of example, but may also be configured as otherwise. The medical injection system 10 may be used to inject a drug, vitamins, a vaccine or any other type of medical solution to a patient or subject for the purpose of medical treatment or clinical testing, etc.

The medical injection system 10 includes a container 20 and a tip cap assembly 40. The container 20 has an elongated barrel 21 and a flange 22 extending radially outwardly from the proximal end of the barrel 21. The barrel 21 extends along the axis A and defines a reservoir 26 in which medical solution to be injected to a patient is stored. The barrel 21 may have a cylindrical shape centered on axis A, but also have other shapes as necessary. The container 20 also has a tip 23 which projects distally from the barrel 21. The tip 23 and the barrel 21 are connected to each other by a shoulder portion 24 which extends from the distal end of the barrel 21 and has a diameter which gradually decreases toward the tip 23.

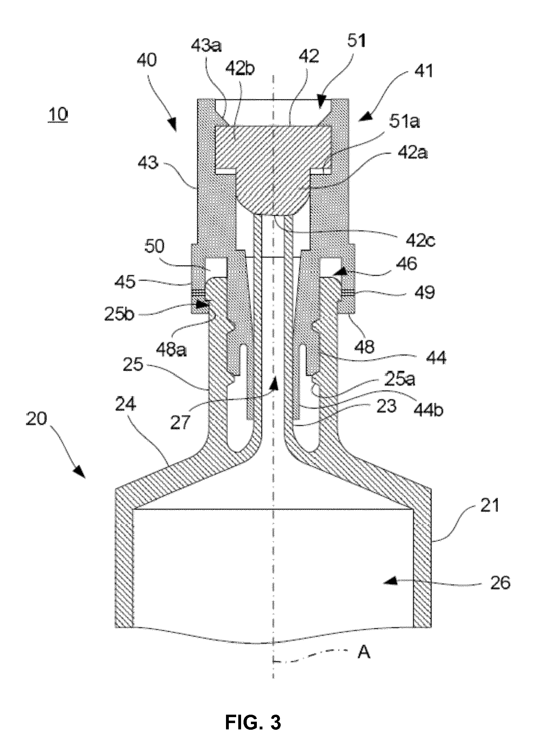

As will appear more clearly in Figure 3, the tip 23 has a conical shape with a diameter smaller than that of the barrel 21. The tip 23 is provided with a fluid passageway 27 extending axially (along the axis A) and throughout the tip 23. The tip 23 may be configured to be connected to an additional element which is not shown in the Figures, e.g., a needle hub or intravenous (IV) line, thereby allowing the medical solution contained in the reservoir 26 to be expelled and further injected into an intended target through the fluid passageway 27.

The container 20 also includes a collar 25 distally extending substantially parallel to the tip 23 from the shoulder portion 24. The collar 25 has a tubular shape with a diameter smaller than that of the barrel 21 but greater than that of the tip 23. The collar 25 surrounds the tip 23 and defines an annular space therebetween for receiving an inner skirt 44 of the tip cap assembly 40. In an embodiment, the tip 23 may extend distally to reach a position farther than the distal end of the collar 25.

In an embodiment, the collar 25 may be integrally formed with the remaining portion of the container 20, i.e., the tip 23, the shoulder portion 24, and the barrel 21. In such a case, the collar 25 may be formed together with the container 20, e.g., by way of injection molding. Alternatively, the collar 25 may be a separate element from the tip 23 or the shoulder portion 24, and fixedly attached to the shoulder portion 24 by screwing, clipping, welding, or applying adhesive or any other known attaching means. The integrally-formed collar 25 is advantageous in particular when the collar 25 has small dimensions since the collar 25 can be easily formed together with the container 20.

The collar 25 has an internally threaded surface 25a on the inner surface facing the external surface of the tip 23 so as to securely receive the tip cap assembly 40 as further described below. In addition, after opening of the tip cap assembly 40, the threaded surface 25a of the collar 25 also allows the connection of a needle hub or an IV line.

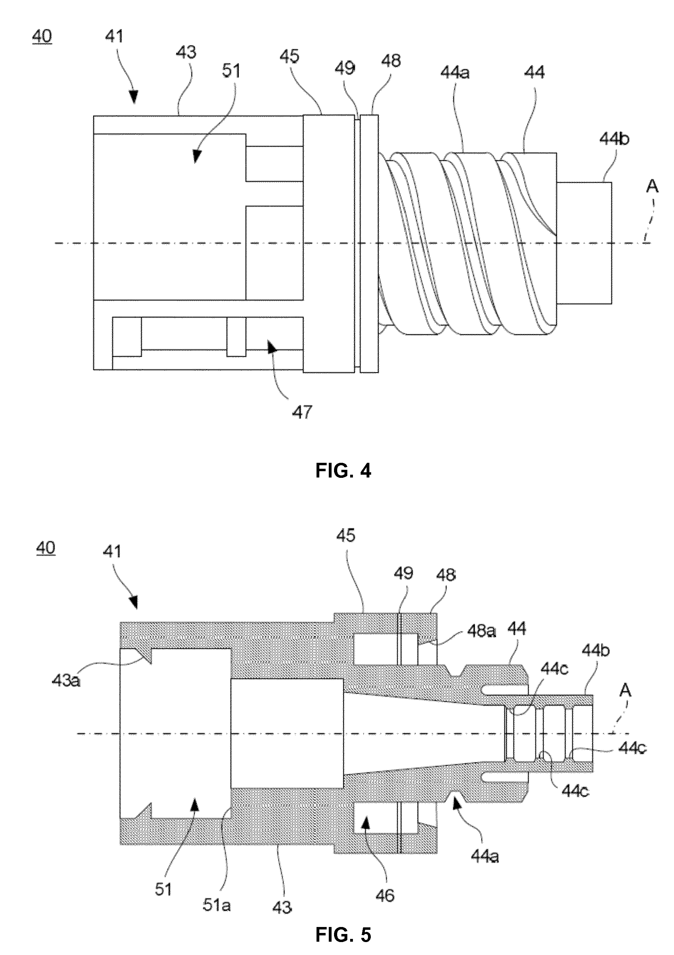

Also with reference to Figures 4 and 5, the detailed configuration of the tip cap assembly 40 will be described below. The tip cap assembly 40 includes a rigid outer cap 41 and an elastomeric inner cap 42 arranged into the outer cap 41. The outer cap 41 is configured such that the tip cap assembly 40 can be securely attached to the container 20 and at the same time it can be easily detached from the container 20 in order to prepare the medical injection system 10 for use. The outer cap 41 may be made from a variety of plastic materials, including but not limited to polypropylene, polyethylene, polyvinylchloride, polystyrene, polycarbonate or a copolymer such as acrylonitrile butadiene styrene or styrene acrylonitrile.

The inner cap 42 is, on the other hand, made of flexible materials which are easily deformable compared to the outer cap 41, such materials including but not limited to natural rubber, acrylate-butadiene rubber, cis-polybutadiene, chloro or bromobutyl rubber, chlorinated polyethylene elastomers, polyalkylene oxide polymers, ethylene vinyl acetate, fluorosilicone rubbers, hexafluoropropylene-vinylidene fluoride-tetrafluoroethylene terpolymers, butyl rubbers, polyisobutene, synthetic polyisoprene rubber, silicone rubbers, styrene- butadiene rubbers, tetrafluoroethylene propylene copolymers, thermoplastic-copolyesters, thermo-plastic elastomers, or the like or any combination thereof. Thanks to the elastic nature of the inner cap 42, the tip cap assembly 40 provides a sufficient sealing effect to reliably close the fluid passageway 27 of the container 20 prior to intended use or during storage.

The outer cap 41 includes a cylindrical hollow body 43, an inner skirt 44 extending proximally from the body 43 and an outer skirt 45 extending proximally from the body 43 and around the inner skirt 44. The body 43 defines a cavity 51 which opens both on the distal end and the proximal end. The cavity 51 has a greater diameter on its distal end than on its proximal end so as to form a stepped portion 51 a at an intermediate position between the distal end and the proximal end.

Referring to Figure 3, the inner cap 42 has a first portion 42a on its proximal side and a second portion 42b on its distal side. The second portion 42b is sized so as to be greater than the first portion 42 such that the first portion 42a extends proximally beyond the stepped portion 51 a and the second portion 42b rests against the stepped portion 51 a.

The first portion 42a has a proximal surface 42c at the proximal end. The proximal surface 42c extends over an area greater than the cross-section area of the fluid passageway 27 defined in a direction perpendicular to the axis A. The proximal surface 42c may be substantially flat or slightly curved. However, as long as the inner cap 42 can sufficiently reliably close the fluid passageway 27 by maintaining tightness between the proximal surface 42c and fluid path 27 through the tip 23 thanks to the compression of the inner cap 42 onto the tip 23, and also prevent from external contamination of the tip 23 with the aid of the radial clamping between the first portion 42a and the body 43 of the outer cap 41, the proximal surface 42c may be configured to have any given shape.

The inner cap 42 has a shape substantially identical to the shape of the cavity 51, but it is sized so as to be slightly greater than the cavity 51. Accordingly, when the inner cap 42 is placed within the cavity 51, the inner cap 42 is in a compressed state so as to be urged against the wall of the cavity 51, and therefore it is ensured that the inner cap 42 is not dislocated from its position in order to provide a reliable sealing effect on the fluid passageway 27.

In an embodiment, the outer cap 41 may be provided with retaining clips 43a on the inner circumferential surface of the outer cap 41 near or at the distal end of the outer cap 41. The retaining clips 43a may be formed so as to be spaced apart from each other along the inner circumference of the outer cap 41. The retaining clips 43a may be arranged diametrically opposite to each other. The retaining clips 43a are engageable with the second portion 42b of the inner cap 42, so as to maintain the inner cap 42 in place and prevent the inner cap 42 from slipping out of the cavity 51.

The outer cap 41 may have one or more openings 47 (see Figure 4) on its circumferential surface through which the cavity 51 is exposed to the outside of the outer cap 41. Such openings 47 may allow part of the inner cap 42 to stick out of the outer cap 41 through the openings 47, whereby the inner cap 42 can provide an even greater sealing effect on the fluid passageway 27. In an embodiment, the openings 47 may be formed at diametrically opposite positions.

The inner skirt 44 and the outer skirt 45 of the outer cap 41 are substantially concentric with each other and define an annular groove 46 therebetween. In an embodiment, the inner skirt 44 extends beyond the outer skirt 45 in the proximal direction. In this case, the depth of the groove 46 depends on the length of the outer skirt 45 from the cylindrical hollow body 43.

The inner skirt 44 has an externally threaded surface 44a which is designed to cooperate with the internally threaded surface 25a of the collar 25. The inner skirt 44 also has an extension 44b branching off therefrom in the proximal direction. The extension 44b is designed to be engaged with the tip 23 of the container 20. Referring to Figure 5, the extension 44b has internal ridges 44c on its inner surface. The internal ridges 44c are in direct contact with the outer surface of the tip 23 in order to provide a gripping effect for reliable engagement between the inner skirt 44 and the tip 23. This further provides tightness between the tip cap assembly 40 and the tip 23, thereby preventing external contamination of the tip 23 and ultimately of the drug container 20.

The outer skirt 45 has a proximal member 48 at the distal end thereof. The proximal member 48 is provided with a protrusion 48a which extends radially inwardly from the proximal member 48. The protrusion 48a is configured so as to be engaged with the recess 25b of the collar 25. Due to the engagement between the protrusion 48a and the recess 25b, the outer skirt 45 and therefore the tip cap assembly 40 can be retained at a predetermined position relative to the container 20. Conversely, the protrusion could be on the collar and the recess could be on the proximal member.

If the protrusion is not properly engaged in the recess, the outer skirt is elastically deformed radially and this deformation can be detected visually, e.g. using an optical control system used in the manufacturing line. It is thus possible to control that the tip cap assembly has been correctly mounted onto the container.

In an embodiment, the protrusion 48a may be in the form of a barb which extends distally from the proximal member 48. The barb provides secure engagement with the recess 25b of the collar 25 once the tip cap assembly 40 is fully assembled to the container 20, while facilitating the assembly of the tip cap assembly 40 onto the container 20.

As illustrated in Figures 3 to 5, the proximal member 48 may be connected to the outer skirt 45 with a weakened portion 49. The weakened portion 49 has a fragile structure configured to easily break under mechanical stress. The weakened portion 49 may be a ring element having a slight contact area with the outer skirt 45 or the proximal member 48. The weakened portion 49 may be integrally formed with the proximal member 48 and the outer skirt 45. In an alternative embodiment, the weakened portion 49 may be a separate element from the proximal member 48 and/or the outer skirt 45 and later attached therebetween by means of welding, for example.

The tip cap assembly 40 is designed to close the fluid passageway 27 of the container 20, in particular by the inner cap 42. When the tip cap assembly 40 is assembled onto the container 20 to close the fluid passageway 27, the tip cap assembly 40 is screwed onto the container 20 thanks to the externally threaded surface 44a of the inner skirt 44 with the internally threaded surface 25a of the collar 25. In particular, the tip cap assembly 40 is screwed onto the container 20 until the protrusion 48a of the tip cap assembly 40 is engaged with the recess 25b of the collar 25.

Figure 3 illustrates the medical injection system 10 in which the tip cap assembly 40 is fully assembled onto the container 20 (the protrusion 48a is engaged within the recess 25a) and the fluid passageway 27 is closed by the inner cap 42. As apparent from this configuration, a longitudinal gap 50 between the distal end of the annular groove 46 and the distal end of the collar 25. The gap 50 can be formed, for example, by lengthening the threaded portion of the inner skirt 44.

Thanks to the gap 50 created between the tip cap assembly 40 and the collar 25, the force applied on the tip cap assembly 40 in order to screw it onto the container 20 can be prevented from directly transmitting to the collar 25. Therefore, crack formation on the collar 25 and the container 20 due to mechanical stress generated during the assembly of the medical injection system 10 can be prevented.

In a fully or partially automated manufacturing line, the tip cap assembly may be screwed onto the container by using a machine. However, some containers and/or tip cap assemblies may be slightly different in size due to the individual differences. As the individual differences are not taken into consideration, the machine applies a predetermined torque to assemble the tip cap assembly onto the container, and in some cases, crack formation may occur in the injection system during the assembly process.

However, according to the embodiment described above, the collar 25 is not subject to the direct transmission of the force, thereby preventing the collar 25 from being damaged or broken.

The engagement between the protrusion 48a and the recess 25a provides a user with a tactile indication, by which a user would get the indication that the tip cap assembly 40 is in a fully-assembled state and at a correct position relative to the container 20. This can prevent a user from accidentally applying excessive force to the tip cap assembly 40 in an attempt to assemble the medical injection system 10.

In an alternative embodiment, the collar 25 may be provided with a protrusion and the outer skirt 45 may be provided with a recess designed to be engaged with the protrusion of the collar 25. As long as it is ensured that the outer skirt 45 is engaged with the collar 25 at a predetermined position relative to each other, the outer skirt 45 and the collar 25 may have other types of complementary structures.

In an embodiment, the proximal member 48 connected to the outer skirt 45 via the weakened portion 49 may serve as a tamper evidence means. With this structure, a user of the injection system 10 can easily detect if the injection system 10 is already opened prior to intended use.

## Claims

1. Tip cap assembly (40) adapted to close a fluid passageway (27) extending through a tip (23) of a container (20) of a medical injection system (10), the tip cap assembly (40) comprising:
a rigid outer cap (41) comprising an inner skirt (44) extending proximally and having an outer threaded surface (44a), and
an elastomeric inner cap (42) arranged within the outer cap (41), the inner cap (42) having a protrusion (42a) extending proximally and adapted to close the fluid passageway (27),
the tip cap assembly (40) being **characterized in that**:
the outer cap (41) further comprises an outer skirt (45) extending proximally and around the inner skirt (44) so as to define an annular groove (46) between the inner skirt (44) and the outer skirt (45), and that
the outer skirt (45) comprises a proximal member (48) having a projection (48a) extending inwardly or a recess depressed outwardly.

2. Tip cap assembly (40) according to claim 1, wherein the proximal member (48) is connected to the outer skirt (45) by a weakened portion (49).

3. Tip cap assembly (40) according to claim 2, wherein the proximal member (48), the outer skirt (45) and the weakened portion (49) are integrally formed with each other.

4. Tip cap assembly (40) according to any one of claims 1 to 3, wherein the inner skirt (44) extends beyond the outer skirt (45) in a proximal direction.

5. Medical injection (10) system comprising:
a container (20) including a longitudinal barrel (21), a tip (23) projecting distally from the barrel (21) and provided with a fluid passageway (27) extending through the tip (23), and a collar (25) extending around the tip (23), the collar (25) having an internal threaded surface (25a) facing an external surface of the tip (23), and
a tip cap assembly (40) according to any one of claims 1 to 4 which is screwed onto the collar (25) in cooperation between the outer threaded surface (44a) of the outer cap (41) and the internal threaded surface (25a) of the collar (25) so as to close the fluid passageway (27), wherein
the proximal member (48) of the outer skirt (45) retains the outer cap (41) onto the collar (25) at a predetermined position relative to each other, and
a longitudinal gap (50) is provided between a distal end of the collar (25) and a bottom of the groove (46) when the outer cap (41) is retained onto the collar (25) by the proximal member (48).

6. Medical injection system (10) according to claim 5, **characterized in that** the collar (25) has a complementary shape to the projection (48a) or to the recess formed on the proximal member (48) of the outer skirt (45).

7. Medical injection system (10) according to claim 6, **characterized in that** the collar (25) has a recess (25b) or protrusion engaged with the protrusion (48a) or the recess of the proximal member (48).

8. Medical injection system (10) according to any one of claims 5 to 7, **characterized in that** the collar (25) and the container (20) are integrally formed with each other.

9. Medical injection system (10) according to any one of claims 5 to 8, **characterized in that** an inner surface of the inner skirt (44) of the outer cap (41) is in contact with the external surface of the tip (23) of the container (20).

10. Medical injection system (10) according to any one of claims 5 to 9, **characterized in that** the proximal member (48) has an annular shape around the collar (25).

11. Process for producing a medical injection system (10), the medical injection system (10) comprising a container (20) formed with a fluid passageway (27) extending through a distal tip (23) of the container (20) and a tip cap assembly (40) adapted to close the fluid passageway (27), the container (20) comprising a collar (25) extending around the tip (23),
the tip cap assembly (40) comprising an inner skirt (44) extending proximally and an outer skirt (45) extending around the inner skirt (44) so as to form an annular groove (46) between the inner skirt (44) and the outer skirt (45), the process comprising:
screwing the tip cap assembly (40) onto the collar (25) such that the collar (25) is introduced to the groove (46) until the tip cap assembly (40) reaches a predetermined position relative to the collar (25) so as to form a longitudinal gap (50) between a distal end of the collar (25) and a bottom of the groove (46).
